# EUROPÄISCHE PATENTSCHRIFT

(11) **EP 3 166 538 B1**
(45) Veröffentlichungstag und Bekanntmachung des Hinweises auf die Patenterteilung: **27.03.2019**
(21) Anmeldenummer: 15734637.0
(22) Anmeldetag: 01.07.2015
(51) Int. Cl.: A61F 2/34, A61L 27/10, A61L 27/56, C04B 41/87

(54) **VOLLKERAMISCHE HÜFTGELENKSPFANNE MIT PORÖSER RÜCKSEITIGER MANTELFLÄCHE**
ENTIRELY CERAMIC ACETABULUM HAVING A POROUS REAR OUTER SURFACE
COTYLE PROTHÉTIQUE EN CÉRAMIQUE INTÉGRALE POURVU D'UNE GARNITURE POREUSE SUR SA FACE ARRIÈRE

(30) Priorität: 09.07.2014 DE 102014213362
(43) Veröffentlichungstag der Anmeldung: 17.05.2017
(73) Patentinhaber: CeramTec GmbH, 73207 Plochingen (DE)
(72) Erfinder: PREUSS, Roman, 73230 Kirchheim unter Teck (DE); RASCHKE, Marita, 71229 Leonberg (DE); STRÖTGEN, Peter, 78216 Königsfeld (DE); WEISS, Tobias, 73061 Ebersbach (DE)
(74) Vertreter: Fehrenbacher, Eckhard Anton
(86) Internationale Anmeldenummer: PCT/EP2015/064928
(87) Internationale Veröffentlichungsnummer: WO 2016/005238

(56) Entgegenhaltungen:
- WO-A1-01/72664
- DE-A1-102011 005 424
- DE-A1-102013 214 657
- GB-A- 2 210 363

## Beschreibung

Die Erfindung betrifft eine Hüftgelenkspfanne für eine Hüftgelenksprothese mit einer inneren Kavität und einer rückseitigen knochenzugewandten Mantelfläche.

Der aktuelle klinische Stand der Technik sieht keramische Gleitflächen für endoprothetische Hüftgelenkspfannen nur in Kombination mit metallischen Komponenten vor (modulare Systeme). Die metallischen Komponenten (Hüftgelenkspfanne) sind häufig mit einer ebenfalls metallischen osseointegrativen Beschichtung versehen, welche das direkte Einwachsen des Knochens in die Implantatoberfläche ermöglicht. Eine keramische Komponente (Einsatz) stellt dagegen den geringen Verschleiß der Gleitpaarung sicher.

Nachteile der modularen Systeme sind die relative große Wanddicke des Komplettsystems hinsichtlich Knochenresektion, sowie Anwendungsfehler durch verkipptes Einsetzen der Keramikeinsätze in die Metallpfannen, was zum Keramikbruch führen kann.

Eine Reduzierung der Wanddicke der modularen Systeme ist durch eine auftretende Verformung der Hüftgelenkspfanne beim Einschlagen limitiert, in deren Folge das Einsetzen der Keramikkomponente erschwert oder komplett unmöglich werden kann.

Neben der Anwendung der o.g. pfannenseitig modularen Hüftgelenksendoprothesen mit keramischen Gleitflächen gibt es im Markt zusätzlich verschiedene direkt implantierbare, metallische, monolithische Pfannenlösungen (inkl. osseointegrativer Beschichtung) mit metallischer Gleitfläche mit dem Ziel der knochensparenden Operation.

DE 10 2011 005424 A1 zeigt eine keramische Hüftgelenkspfanne für Hüftgelenksprothesen mit einer rückseitigen knochenzugewandten Mantelfläche, die zumindest teilweise porörs und osseointegrativ ausgebildet ist. Die poröse Schicht wird dazu auf den Sinterformkörper der Hüftgelenkspfanne aufgebracht.

Die Anwendung einer direkt implantierbaren vollkeramischen Hüftgelenkspfanne mit integrierter poröser osseointegrativer rückseitiger Mantelfläche stellt eine vorteilhafte Neuerung dar.

Eine derartige metallfreie Hüftgelenkspfanne vereint die Vorteile der knochensparenden Operation mit denen eines biokompatiblen Materials, geringen Verschleißes der Gleitpaarung und hervorragender Osseointegration.

Eine Hüftgelenkspfanne für eine Hüftgelenksprothese mit einer inneren Kavität und einer rückseitigen knochenzugewandten Mantelfläche, ist dadurch gekennzeichnet, dass die Mantelfläche zumindest in Teilbereichen porös und osseointegrativ ausgebildet ist und die Hüftgelenkspfanne ausschließlich aus einem keramischen Material besteht.

Ein erfindungsgemäßes Verfahren zur Herstellung der porösen osseointegrativen Manterfläche der Hüftgelenkspfanne ist dadurch gekennzeichnet, dass die Hüftgelenkspfanne aus einem Sinterformkörper hergestellt wird und auf die Mantelfläche zumindest in Teilbereichen eine poröse osseointegrative Schicht aufgebracht wird.

In der erfindungsgemäßen Ausführung des Verfahrens wird die poröse Mantelfläche durch Aufbringen eines keramischen Schlickers und Platzhaltern (Porenbildner) im Grünzustand und Ausbrennen der Platzhalter beim Sintern hergestellt. Bevorzugt kann die poröse Schicht durch Schäumen eines Schlickers im Grünzustand und anschließendes Sintern entstehen. Über den Sintervorgang wird eine Verbindung mit der Mantelfläche erreicht und es entsteht eine offenporige Struktur mit zerklüfteter Oberfläche, die das An - und Einwachsen des Knochen vorteilhaft unterstützt.

Diese Art der Beschichtung im Verbund mit der keramischen Hüftgelenkspfanne ist ebenfalls metallfrei und ist dadurch vorteilhaft im Bezug auf allergische Reaktionen.

Der keramische Schlicker besteht aus dem selben Material wie die Hüftgelenkspfanne. Die komplette Hüftgelenkspfanne ist dadurch inklusive Beschichtung komplett frei von Metallen und vorteilhaft im Bezug auf allergische Reaktionen.

Die poröse Mantelfläche kann auch durch Fügen eines dichten Formkörpers mit einem porösen Formkörper im gesinterten Zustand entstehen. Der poröse Formkörper kann durch Schäumen von keramischem Schlicker und Sintern oder Infiltrieren eines porösen Trägermaterials mit Schlicker und Sintern entstehen. Das Fügen wird bevorzugt durch Löten oder Kleben durchgeführt.

Die poröse osseointegrative Mantelfläche kann erfindungsgemäß auch durch 2K-Spritzguß (dichte Phase und poröse oder porenbildende Phase) und Sintern hergestellt werden.

Die poröse Mantelfläche kann bevorzugt durch weitere Beschichtung eine zusätzliche Funktionalität erhalten, wie höhere Integrationsgeschwindigkeit oder antibakterielle Wirkungen. Mit Integrationsgeschwindigkeit ist die Zeit des Einwachsens mit dem Knochen verstanden. Bevorzugt kann dies mit Biogläsern, Hydroxilapatit, funktionalisierten Eiweißen oder Hydrogelen erreicht werden.

Die erfindungsgemäße Hüftgelenkspfanne besteht vollständig aus einer Keramik und kann durch die poröse Mantelfläche direkt implantiert werden. Der Knochen wächst schnell in die poröse Mantelfläche ein, so dass ein fester Sitz schon bald nach der Operation gewährleistet ist. Bei der Implantation wird kein Knochenzement verwendet.

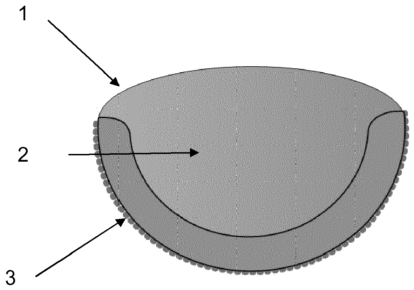

Figur 1 zeigt eine Hüftgelenkspfanne 1 mit einer inneren Kavität 2 und einer rückseitigen knochenzugewandten Mantelfläche 3. Erfindungsgemäß ist die Mantelfläche 3 zumindest in Teilbereichen porös und osseointegrativ ausgebildet. Die Hüftgelenkspfanne 1 besteht ausschließlich aus einer Keramik bzw. aus einem keramischen Material.

## Patentansprüche

1. Verfahren zur Herstellung einer Hüftgelenkspfanne (1) für eine Hüftgelenksprothese mit einer inneren Kavität (2) und einer rückseitigen knochenzugewandten Mantelfläche (3), wobei die Mantelfläche (3) zumindest in Teilbereichen porös und osseointegrativ ausgebildet ist und die Hüftgelenkspfanne (1) ausschließlich aus einem keramischen Material besteht, **dadurch gekennzeichnet, dass** die poröse Mantelfläche (3) durch Aufbringen eines keramischen Schlickers und Platzhaltern (Porenbildner) im Grünzustand und Ausbrennen der Platzhalter beim Sintern hergestellt wird und der keramische Schlicker aus dem selben Material wie die Hüftgelenkspfanne besteht.

2. Verfahren nach Anspruch 1, **dadurch gekennzeichnet, dass** die poröse Mantelfläche (3) durch weitere Beschichtung eine zusätzliche Funktionalität erhält, wie höhere Integrationsgeschwindigkeit oder antibakterielle Wirkungen.

3. Verfahren nach Anspruch 2, **dadurch gekennzeichnet, dass** die poröse Mantelfläche (3) mit Biogläsern, Hydroxilapatit, funktionalisierten Eiweißen oder Hydrogelen beschichtet wird.

## Claims

1. Method for producing an acetabulum (1) for a hip joint prothesis, comprising an inner cavity (2) and a rear outer surface (3) which faces the bone, the outer surface (3) being porous and osseointegrated at least in portions, and the acetabulum (1) consisting exclusively of a ceramic material, **characterized in that** the porous outer surface (3) is produced by applying a ceramic slip and placeholders (pore-forming agents) in the green state and burning off the placeholders during sintering, and the ceramic slip consists of the same material as the acetabulum.

2. Method according to claim 1, **characterized in that** the porous outer surface (3) acquires additional functionality by means of further coating, such as higher integration speed or antibacterial effects.

3. Method according to claim 2, **characterized in that** the porous outer surface (3) is coated with bioglasses, hydroxylapatite, functionalized proteins, or hydrogels.

## Revendications

1. Procédé de fabrication d'un cotyle (1), destiné à une prothèse de hanche, qui comporte une cavité interne (2) et une surface d'enveloppe arrière (3) orientée vers l'os, la surface d'enveloppe (3) étant conçue, au moins par endroits, pour être poreuse et pour permettre l'ostéointégration, et le cotyle (1) est constitué exclusivement un matériau céramique, **caractérisé en ce que** la surface d'enveloppe poreuse (3) est fabriquée par application d'une barbotine céramique et d'éléments de remplissage (agents formateurs de pores) à l'état brut et par combustion des éléments de remplissage lors du frittage, et la barbotine céramique est constituée du même matériau que le cotyle.

2. Procédé selon la revendication 1, **caractérisé en ce qu'**un revêtement supplémentaire confère à la surface d'enveloppe poreuse (3) une autre fonctionnalité, telle qu'une vitesse d'intégration plus élevée ou des effets antibactériens.

3. Procédé selon la revendication 2, **caractérisé en ce que** la surface d'enveloppe poreuse (3) est revêtue de bioverres, d'hydroxylapatite, de protéines fonctionnalisées ou d'hydrogels.
